# DEMANDE DE BREVET EUROPEEN

(11) **EP 2 532 371 A1**
(43) Date de publication de la demande: **12.12.2012**
(21) Numéro de dépôt: 12170268.2
(22) Date de dépôt: 31.05.2012
(51) Int. Cl.: A61L 9/014, A61L 9/16, B01D 53/04, B65D 81/26, F25D 17/04

(54) **Contenant de stockage à capacité d'assainissement de gaz**

(30) Priorité: 09.06.2011 FR 1101774
(71) Demandeur: Kotchenko-Heitz, Nadia, 77910 Germigny-l'Evêque (FR)
(72) Inventeur: Kotchenko-Heitz, Nadia, 77910 Germigny-l'Evêque (FR)
(74) Mandataire: Novagraaf Technologies

(57) **Abrégé**

Est concerné un contenant (1) de stockage placé dans un milieu gazeux ambiant (3) pour son assainissement. Il comprend un réservoir (5) adapté à contenir une argile sèche, un opercule (7) poreux, perméable au gaz, sans se laisser traverser par le matériau absorbant (13), et une pellicule(9) et/ou un couvercle (11) non poreux et imperméables, recouvrant l'opercule mais qui sont alors à retirer pour permettre l'assainissement.

## Description

L'invention concerne un contenant de stockage placé dans un milieu gazeux ambiant à fin d'assainissement.

A partir de US4624366 est connu un contenant comprenant :
- un réservoir adapté à contenir un matériau absorbant, sec comprenant une argile sèche, le réservoir présentant une ouverture,
- un opercule poreux fermant l'ouverture, l'opercule étant perméable au gaz, sans se laisser traverser par le matériau absorbant, afin de laisser passer de l'extérieur vers l'intérieur du réservoir du gaz ambiant à assainir pour qu'il y entre en contact avec le matériau absorbant et laisser du gaz assaini ressortir à travers l'opercule,
- une pellicule et/ou un couvercle non poreux et imperméables, recouvrant l'opercule de façon amovible jusqu'à utilisation à fin d'assainissement, cette pellicule et ce couvercle étant alors à retirer pour découvrir l'opercule.

Il est précisé que, dans le présent texte, l'expression « laisser passer de l'extérieur vers l'intérieur du réservoir du gaz ambiant à assainir pour qu'il y entre en contact avec le matériau absorbant et laisser du gaz assaini ressortir à travers l'opercule » a pour sens non pas nécessairement un filtrage d'air extérieur malodorant par le matériau absorbant à base d'argile, mais plutôt un piégeage et/ou blocage de mauvaises odeurs au sein de ce matériau absorbant.

Sur cette base, le problème concerne :
- la préservation du matériau absorbant jusqu'à (au moins première) utilisation,
- l'efficacité des échanges gazeux pour favoriser l'assainissement,
- l'atteinte d'une praticité d'emploi,
- l'atteinte d'un objectif de prix de revient faible.

Pour atteindre tout ou partie de ces objectifs, il est recommandé en particulier les choix suivants, suite à des essais convaincants :
- un opercule poreux présentant une densité comprise entre 40 et 70 g/m²,
- et/ou un opercule poreux présentant une perméabilité à l'air, à 196 Pa, comprise entre 400 et 500 L/m2/s.

A priori, la liaison séparable entre la pellicule de protection, ou le couvercle, et le réservoir se réalisera à l'endroit d'un rebord saillant vers l'extérieur que présentera avantageusement le réservoir au niveau de son ouverture supérieure fermée (de façon permanente) par l'opercule poreux. On conseille que l'opercule poreux soit une feuille plane recouverte d'une couche d'adhésion thermoscellable au réservoir, leurs matières étant alors compatibles à cet effet, l'opercule poreux étant ainsi scellé périphériquement et de façon définitive audit rebord.

Ainsi, les conditions de fabrication seront aisées et on ne risquera pas de répandre de l'argile en poudre ou granuleux hors du réservoir. En outre, une protection efficace jusqu'à premier emploi du contenant sera favorisée.

Pour favoriser l'atteinte d'un compromis entre l'efficacité des échanges gazeux favorisant l'assainissement et la quantité d'argile nécessaire, on recommande que la quantité d'argile sèche dans le réservoir soit comprise entre 60 et 200g, et de préférence entre 100 et 150g, avec de préférence une surface d'échange, dans le réservoir, entre l'argile et le gaz ambiant à assainir, face au dessous de l'opercule poreux comprise entre 0,0025 et 0,0055 m2.

Pour un tel compromis, des essais ont aussi montré l'avantage de l'utilisation d'un non tissé fibreux pour constituer l'opercule poreux.

Pour favoriser le thermoscellage et l'efficacité des échanges gazeux, on conseille que l'opercule poreux soit cellulosique. En alternative, on propose que l'opercule poreux soit issu de non tissé polymères tels que le polypropylène ou le PET.

Pour éviter que l'utilisateur soit tenté d'arracher l'opercule poreux, une fois celui-ci thermoscellé sur le réservoir, on recommande que cet opercule présente un diamètre extérieur inférieur à celui du rebord du réservoir. On recommande également qu'il soit dépourvu de languette saillante favorisant une prise par les doigts de l'utilisateur.

Pour un compromis entre une bonne herméticité avant emploi, un coût de fabrication réduit et une réelle praticité d'utilisation, on conseille par ailleurs que le contenant comprenne à la fois :
- ladite pellicule, laquelle sera alors imprimée,
- et le couvercle, lequel sera alors fixé en périphérie autour du rebord, à force de façon élastique, en emprisonnant ainsi la pellicule (qui sera sinon volante) entre cette périphérie et ce rebord.

Pour un compromis entre une bonne herméticité avant emploi et l'efficacité recherchée des échanges gazeux, on recommande par ailleurs que l'opercule poreux soit recouvert d'encre sur moins de 25% de sa surface, sur sa face supérieure, laquelle sera alors visible à travers le couvercle (ici prévu), lequel recouvrira la pellicule qui recouvrira alors elle-même entièrement l'opercule qui, lui, fermera l'ouverture.

D'autres caractéristiques et avantages des solutions ici proposées apparaîtront encore dans la description qui suit, faite en référence aux dessins annexés où :
- la figure 1 montre une réalisation privilégiée d'un contenant de stockage, dans ses constituants essentiels,
- la figure 2 montre une vue partielle de dessus de la zone II de la figure 1, avec arrachement et vue plus détaillée figurant l'encre qui recouvrira a priori la face supérieure de l'opercule,
- les figures 3, 4 montrent respectivement l'état opérationnel du contenant de stockage et son état non actif, de rangement.

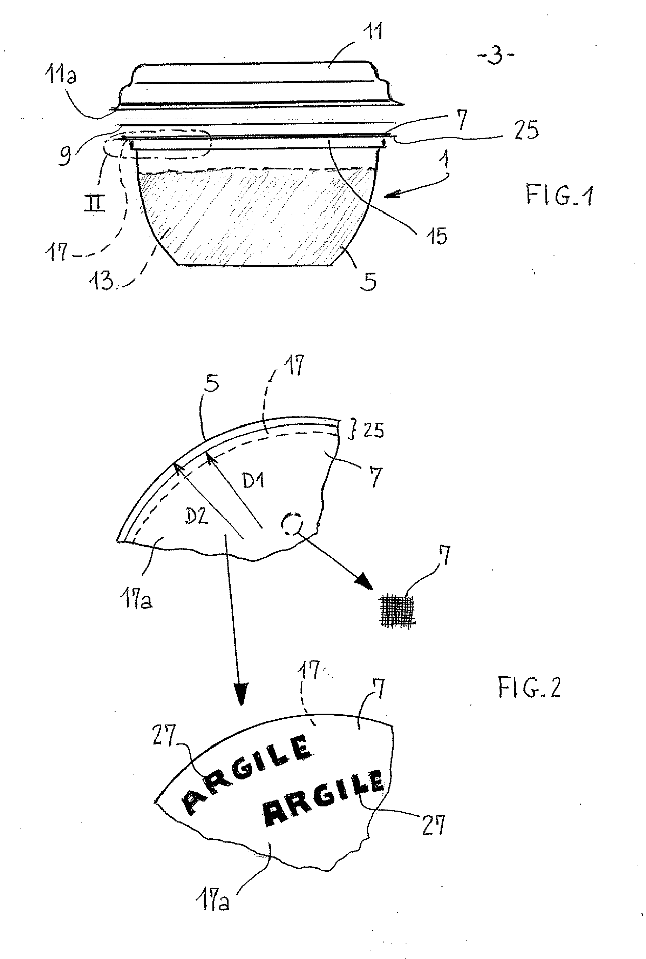

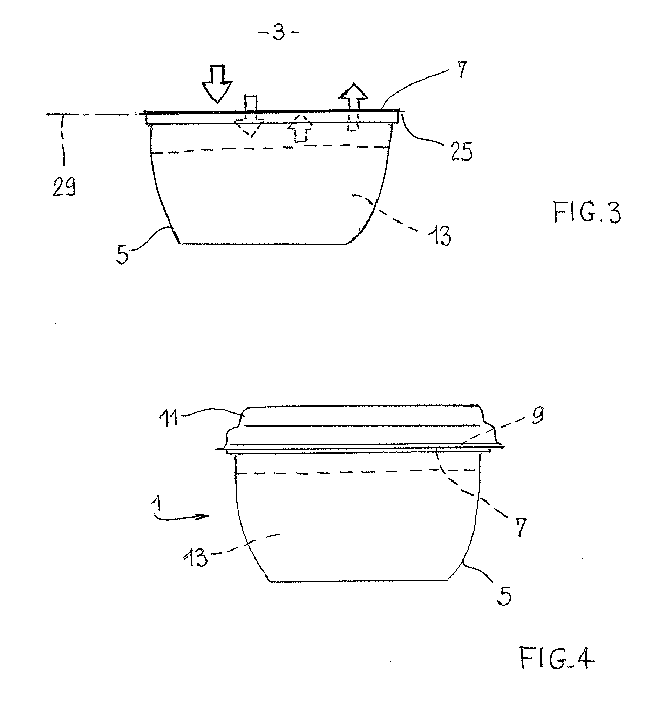

   Figures 1, 4, on voit ainsi un contenant de stockage 1 (ou coupelle) placé dans un milieu gazeux ambiant 3.
   Figure 1 notamment, on constate que ce contenant comprend un réservoir 5, un opercule poreux 7, et, une pellicule 9 et un couvercle 11 non poreux et imperméables (en particulier aux gaz, y compris l'air ambiant).

Le réservoir 5 est adapté à contenir un matériau absorbant 13 sec comprenant une argile sèche. Ce réservoir présente une ouverture supérieure 15.

L'argile sèche sera avantageusement une illite, choisie pour sa qualité purifiante. Qu'elle soit difficile à rincer est ici indifférent.

L'opercule poreux 7 ferme en permanence l'ouverture 15. Cet opercule est perméable aux gaz et ne se laisse pas traverser par le matériau absorbant 13, afin (voir flèches figure 3) de :
- laisser passer de l'extérieur vers l'intérieur du réservoir du gaz ambiant 3 à assainir pour qu'il y entre en contact avec le matériau absorbant,
- et laisser du gaz assaini ressortir à travers l'opercule .

La pellicule et le couvercle, tous deux donc non poreux et imperméables, recouvrent l'opercule 7 de façon amovible jusqu'à utilisation à fin d'assainissement (voir figure 4). S'ils existent ensemble, ils sont tous deux à retirer pour découvrir l'opercule.

Eventuellement, seul le couvercle 11 ou la pellicule 9 pourrait être prévu pour recouvrir et protéger l'opercule poreux 7 de façon amovible pendant les périodes de non utilisation du contenant (figure 4).

Si elle est prévue, la pellicule 9, fine et flexible, sera en papier de 100 à 160 g/m2.

Le couvercle 11, plus rigide, pourra être en Polypropylène, en PVC ou en PET de 200 à 400µm d'épaisseur.

Comme déjà indiqué, on conseille que la quantité d'argile sèche dans le réservoir soit comprise entre 60 et 200g, et de préférence entre 100 et 150g, avec de préférence une surface d'échange, dans le réservoir, entre l'argile et le gaz ambiant à assainir, face au dessous de l'opercule poreux, comprise entre 0,0025 et 0,0055 m2. Le volume de cette zone d'échange 130, sous l'opercule poreux, sera de préférence compris entre 0,025 et 0,055 m3. Quant à la densité de l'opercule poreux, on la préférera alors comprise entre 40 et 70g/m2. La granulométrie du composant granuleux ou pulvérulent 13, typiquement donc (au moins essentiellement) de l'argile, sera entre 60 microns et 3 mm. Ce composant pourra incorporer des huiles essentielles.

De façon de préférence complémentaire, on choisira pour cet opercule une perméabilité à l'air, à 196 Pa, comprise entre 400 et 500 L/m2/s (soit entre 0,4 et 0,5 m3/m2/s ).

Pour s'assurer d'une tenue performante de l'opercule poreux 7 sur le réservoir 5, on recommande que ce dernier présente, autour de l'ouverture 15, un rebord 25 saillant vers l'extérieur.

L'opercule poreux 7 sera alors avantageusement une feuille plane recouverte (en face inférieure) d'une couche 17 d'adhésion thermoscellable au réservoir, leurs matières étant compatibles à cet effet. L'opercule poreux sera ainsi scellé périphériquement et de façon définitive au rebord 25.

De préférence, cet opercule 7 présentera un diamètre extérieur D1 inférieur à celui D2 du rebord 25 du réservoir (figure 2). Ainsi, une fois l'opercule fixé au réservoir, il sera difficile de l'attraper, donc de l'arracher, si un utilisateur est tenté de le faire.

Le rebord 25 du réservoir s'étendant de préférence dans un plan, a priori horizontal, 29, l'opercule s'étendra alors dans ce plan, une fois fixé à ce rebord.

Et, pour ne pas altérer les échanges gazeux ni la capacité de cet opercule 7 à être imprimé, notamment afin d'être attractif en vue de la promotion de ce contenant, il est aussi prévu que la pellicule imperméable et imprimable 9 soit justement imprimée.

Quant au couvercle 11, on le conseille fixé en périphérie 11a autour du rebord 25, à force de façon élastique (liaison clipsée), en emprisonnant alors périphériquement la pellicule 9, qui est sinon volante, entre cette périphérie et ce rebord.

Ainsi, on recommande de ne pas fixer la pellicule 9 à aucun élément, afin de pouvoir aisément la retirer et la mettre en place sur l'opercule poreux 7.

Typiquement, la pellicule 9 sera recouverte sur sa face supérieure par un message ou un dessin attractif, le couvercle 11 étant alors favorablement transparent.

Afin de maintenir attractif l'ensemble, même pellicule 9 retirée, tout en favorisant alors les échanges gazeux, on recommande par ailleurs que l'opercule poreux 7 soit recouvert d'encre 27 (figure 2), sur moins de 25% de sa surface, sur sa (seule) face supérieure 17a, laquelle sera alors favorablement visible à travers le couvercle 11.

Ainsi, le couvercle 11 recouvrira alors la pellicule 9 qui recouvrira elle-même entièrement l'opercule 7 qui fermera l'ouverture 15.

Avantageusement, et comme déjà indiqué, l'opercule poreux 7 sera un non tissé fibreux (voir figure 2). Un opercule poreux 7 cellulosique sera même préféré (voir figure 2).

Ceci favorisera, lors de l'assemblage des éléments du contenant, les manipulations de l'opercule et son positionnement sur le réservoir (pour l'y fixer) par une machine auto ou semi-automatique.

En effet, en cette matière, de tels films, très souples, sont difficiles à manipuler et à positionner, car ils ont tendance à rouler sur eux-mêmes ou à vriller.

Avec des caractéristiques de produits, matières et composants correspondant à ce qui est indiqué ci-avant comme solution préférée, des tests ont été menés, amenant à une efficacité surprenante.

Pour ces tests, des odeurs de réfrigérateurs ont été générées afin d'étudier la capacité des coupelles/contenants à capacité d'absorption ici présenté(e)s.

A cette fin, un réfrigérateur a été rempli des aliments suivants au jour 1 de tests d'olfactométrie :
- 500 g de viande hachée de boeuf non cuite, dans une assiette,
- un filet de poisson truite dans une assiette,
- un chou haché dans un bol ouvert,
- un oignon jaune, tranché, dans une assiette,
- 250 g de fromage à pâte molle et à croûte lavée,
- trois oeufs cuits durs et écrasés dans une assiette.

Le réfrigérateur utilisé était vide, propre et exempt d'odeur particulière. La durée du test était de sept jours pendant lesquels le réfrigérateur est resté fermé. Sa température était ajustée à 6°C. La capacité du réfrigérateur était d'environ 0,5 m3 (résultats toutefois a priori valables pour 0,3 à 1,5 m3).

Les odeurs du réfrigérateur ont été échantillonnées le huitième jour suivant la mise en place des aliments à l'intérieur du réfrigérateur. Un trou percé dans le réfrigérateur a été utilisé pour échantillonner les odeurs. Ainsi, la porte du réfrigérateur n'a pas été ouverte.

La prise d'échantillons d'odeurs s'effectue par un équipement comprenant un poumon sous vide et des sacs échantillons. Un poumon sous vide a pour but de remplir les sacs d'échantillonnage sans avoir à faire circuler l'échantillon à traves une pompe.

Par ailleurs, les seuls matériaux entrant en contact avec l'échantillon sont ceux requis par la norme CEN EN 13725. Les sacs utilisés lors de la prise d'échantillons ont une capacité de 40 litres et sont faits à partir d'un matériau conforme à la norme CEN EN 13725. Ces sacs d'échantillonnage sont à usage unique. Avant leur utilisation, et selon les différents essais, les coupelles servant aux différents traitements sont insérées dans le sac d'échantillonnage avant leur fermeture et les sacs sont entièrement vidés par vacuum et ce, moins d'une heure avant le prélèvement des odeurs. Ils sont ensuite remplis d'air frais filtré et sont vidangés.

Après l'échantillonnage, les sacs sont déposés dans un endroit frais et sombre pour une période de 18 heures pour le premier test et 48 heures pour les tests suivants.

Lors de l'échantillonnage, une alternance entre les sacs d'échantillonnages avec des types de traitements différents a été observée afin que les odeurs du réfrigérateur soient bien distribuées dans les coupelles testées. Un débitmètre a été utilisé afin de restreindre le volume d'air vicié par sac d'échantillonnage.

Plusieurs coupelles/contenants ont été utilisé(e)s, tou(te)s respectant les caractéristiques présentées avant.

L'analyse des échantillons a été effectuée à l'aide d'un olfactomètre dynamique et de panélistes expérimentés.

Les résultats de ce rapport sont principalement présentés sous forme d'unité d'odeur par mètre cube (UO/m3) . Par définition, un UO/m3 correspond au seuil de perception du nez humain pour 50% de la population.

Les tableaux suivants présentent les résultats obtenus lors de ces analyses (quand l'opercule est prévu, il est du type préféré précité).

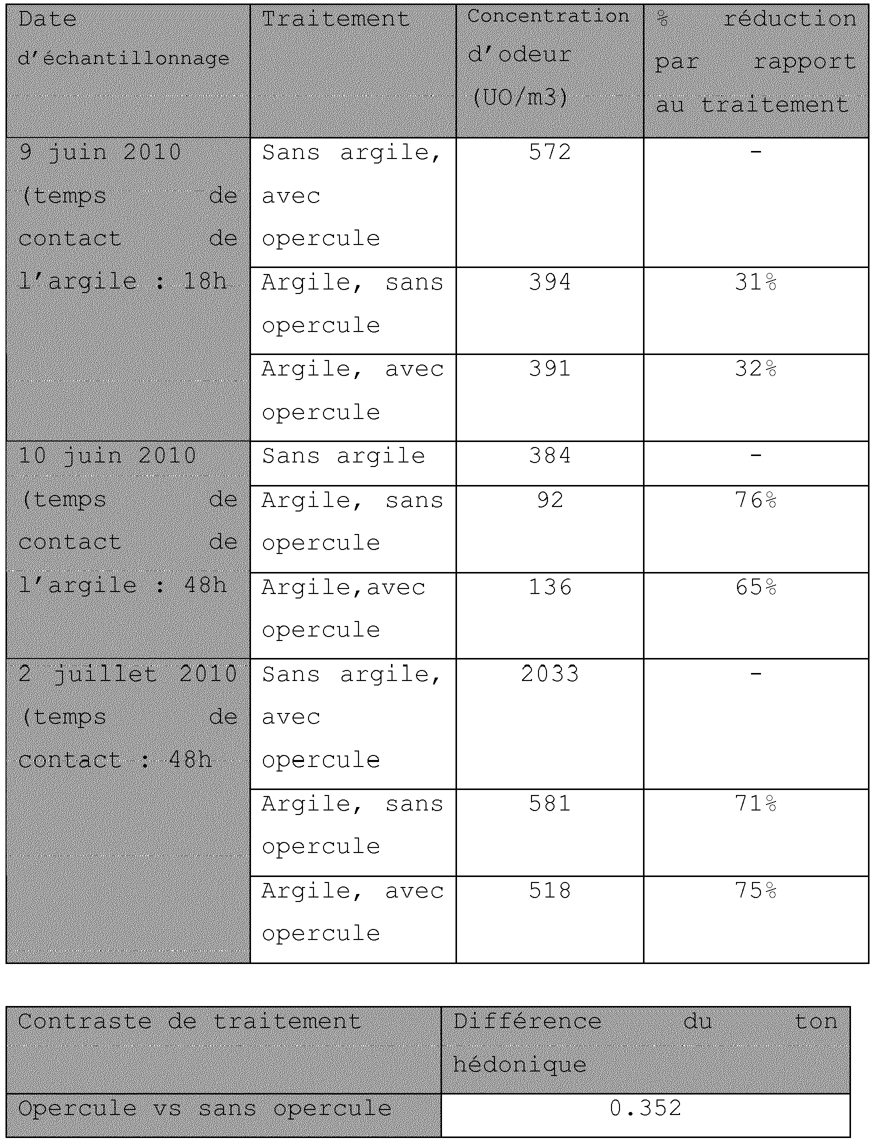

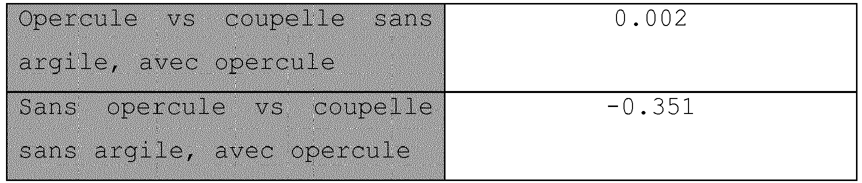

Le ton hédonique est un index de nuisance d'une odeur. Il est échelonné de 0 à 10. 0 est une odeur tolérable et 10 une odeur très désagréable.

Il est possible de conclure que les coupelles d'argile, avec l'opercule utilisé (voir descriptif précédent) permettent de diminuer la concentration de l'odeur de réfrigérateur, mais ne permettent pas de transformer (ou masquer) l'odeur de réfrigérateur pour la rendre plus agréable. Ceci prouve que l'argile a agi comme un absorbant d'odeur et non comme un masquant. Concernant l'opercule 7, on en déduit donc qu'il empêche le composant argileux 13 de se répandre hors du réservoir 5, sans nuire aux échanges gazeux et/ou à l'assainissement.

## Revendications

1. Contenant de stockage placé dans un milieu gazeux ambiant, **caractérisé en ce que** ce contenant (1) comprend :
- un réservoir (5) adapté à contenir un matériau absorbant (13) sec, le réservoir (5) présentant une ouverture (15),
- un opercule poreux (7) fermant l'ouverture (15), l'opercule étant perméable au gaz, sans se laisser traverser par le matériau absorbant (13), afin de laisser passer de l'extérieur vers l'intérieur du réservoir (5) du gaz ambiant (3) à assainir pour qu'il y entre en contact avec le matériau absorbant (13) et laisser du gaz assaini ressortir à travers l'opercule (7),
- une pellicule (9) et/ou un couvercle (11) non poreux et imperméables, recouvrant l'opercule (7) de façon amovible jusqu'à utilisation à fin d'assainissement, cette pellicule (9) et ce couvercle (11) étant alors à retirer pour découvrir l'opercule,
**caractérisé en ce que** le réservoir contient une argile sèche et l'opercule poreux (7) présente une densité comprise entre 40 et 70 g/m2, et/ou une perméabilité à l'air, à 196 Pa, comprise entre 400 et 500 L/m2/s.

2. Contenant (1) de stockage selon la revendication 1, **caractérisé en ce que** la quantité d'argile sèche dans le réservoir (5) est comprise entre 60 et 200g, avec une surface d'échange, dans le réservoir (5), entre l'argile et le gaz ambiant (3) à assainir, face au dessous de l'opercule poreux (7) comprise entre 0,0025 et 0,0055m2.

3. Contenant (1) de stockage selon l'une des revendications 1 ou 2, **caractérisé en ce que** l'opercule poreux (7) est un non tissé fibreux.

4. Contenant (1) de stockage selon la revendication 3, **caractérisé en ce que** l'opercule poreux (7) est cellulosique.

5. Contenant (1) de stockage selon la revendication 3, **caractérisé en ce que** l'opercule poreux (7) est issu de non tissé polymères.

6. Contenant (1) de stockage selon l'une au moins des revendications précédentes, **caractérisé en ce que** le réservoir (5) présente une collerette (25) et l'opercule poreux (7) est une feuille plane recouverte d'une couche d'adhésion thermoscellable au réservoir, leurs matières étant compatibles à cet effet, l'opercule poreux (7) étant ainsi scellé périphériquement et de façon définitive à ladite collerette(25).

7. Contenant (1) de stockage selon la revendication 6, **caractérisé en ce que** l'opercule poreux (7) présente un diamètre extérieur (D1) inférieur à celui (D2) de la collerette (25) du réservoir (5).

8. Contenant (1) de stockage selon la revendication 6, **caractérisé en ce qu'**il comprend ladite pellicule (9), laquelle est imprimée, et le couvercle (11), lequel est fixé en périphérie autour de la collerette (25), à force de façon élastique, en emprisonnant alors la pellicule (9), qui est sinon volante, entre cette périphérie et cette collerette (25).

9. Contenant (1) de stockage selon la revendication 7 et/ou 8, **caractérisé en ce qu'**il comprend ladite pellicule (9) et le couvercle (11), l'opercule poreux (7) étant recouvert d'encre (27) sur moins de 25% de sa surface, sur sa face supérieure, laquelle est visible à travers le couvercle (11), pellicule retirée, le couvercle recouvrant sinon cette pellicule (9) qui recouvre elle-même entièrement l'opercule (7) qui ferme l'ouverture (15).
